# EUROPEAN PATENT SPECIFICATION

(11) **EP 3 685 399 B1**
(45) Date of publication and mention of the grant of the patent: **15.07.2026**
(21) Application number: 18765374.6
(22) Date of filing: 24.08.2018
(51) Int. Cl.: G16H 40/67, G16H 20/17, G16H 20/10, G16H 40/63

(54) **SYSTEM AND METHOD FOR PROVIDING HOMECARE TO A PATIENT**
SYSTEM UND VERFAHREN ZUR BEREITSTELLUNG VON HEIMVERSORGUNG FÜR EINEN PATIENTEN
PROCÉDÉ ET APPAREIL DE FOURNITURE DE SOINS À DOMICILE À UN PATIENT

(30) Priority: 18.09.2017 EP 17306199
(43) Date of publication of application: 29.07.2020
(73) Proprietor: Fresenius Vial SAS, 38590 Brézins (FR)
(72) Inventor: MERMET, Emeric, 38950 Saint Martin Le Vinoux (FR)
(74) Representative: Fresenius Kabi Deutschland GmbH
(86) International application number: PCT/EP2018/072848
(87) International publication number: WO 2019/052795

(56) References cited:
- US-A1- 2004 176 984
- US-A1- 2009 294 521
- US-A1- 2016 000 997
- US-A1- 2017 230 907

## Description

The invention relates to a system for providing homecare to a patient according to claims 1-9.

A system of this kind comprises a medical pump device which is programmable to carry out an administration operation for administering a medical fluid to a patient. The medical pump device herein comprises a pumping mechanism for administering the medical fluid, a storage device for storing programming information, a processor device for controlling operation of the pumping mechanism according to the programming information, and a reader device for reading identification information of an identification mark of a fluid container containing a medical fluid.

There generally is a desire to provide healthcare to a patient at his/her home. This may help to provide for an increased comfort for the patient and may allow the patient to live, potentially, a regular life.

Providing care to a patient at his/her home however comes with the challenge that patients may live far away from a healthcare professional, such as a physician or a nurse in a hospital or in private practice. In addition, patients may live far apart from one another, such that the checking of patients by for example a nurse may require large travel distances to visit one patient after the other. For this reason, patients may be required to operate their medical pump devices autonomously. Hence, patients may have to set up and start a delivery operation on their own according to a prescription which they have obtained from a healthcare professional. This however does not alleviate the need for a regular supervision and checking by a healthcare professional, because if a patient does not follow a prescribed treatment closely or if malfunctions during a delivery operation occur without appropriate action, this may lead to severe effects, potentially requiring a re-hospitalization of the patient.

Homecare patients may be subject to different treatments, including for example infusions or the enteral feeding by administering a nutritional solution. To carry out an administration operation, a patient may be required to program the medical pump device, for which the patient conventionally must specify a medical fluid to be administered and in addition must specify particular administration parameters, such as the dosage, the dose rate and a delivery mode (including for example a continuous administration or a time varying administration scheme such as a ramp-up or the like). Such programming may be difficult and time-consuming for the patient and in addition may be prone to errors.

Typically, the patient is required to program the medical pump device according to prescription information he receives from a medical caregiver such as a nurse, such prescription information specifying the dosage, dose rate and delivery mode for the particular medical fluid to be administered. If the medical pump device, such as a volumetric infusion pump, includes a so called drug library, the patient, when programming the medical pump device, may choose from different, predfined medical fluids such as medication solutions or enteral feeding solutions, the drug library for example specifying boundaries for administration parameters such as the dose rate or the like. Nevertheless, even if a drug library is installed on the medical pump device, the user must choose from a large number of different entries relating to different medical fluids in order to program the medical pump device, which is cumbersome and potentially prone to errors.

There is a need to make the programming of a medical pump device as easy as possible for a user, in particular a patient, in particular in a homecare environment. There furthermore is a desire to be able to improve traceability of administered medical solutions and to increase safety by ensuring that an actual administration operation is compliant with a prescription. US2004/176984 discloses a patient medication IV delivery pump with wireless communication to a hospital information management system. US2009/294521 discloses an interactive medication container labeling. US2016/000997 discloses a management of pending medication orders.

It is an object of the invention to provide a system for providing homecare to a patient which in an easy and efficient, yet reliable way allow a patient to program a medical pump device for carrying out an administration operation.

This object is achieved by means of a system comprising the features of claims 1-9.

### Summary of the invention

The invention is set out in the appended claims.

### Summary of related disclosure

Accordingly, the communication device is constituted to transmit a prescription data set comprising programming information associated with a particular medical fluid to the medical pump device, and the medical pump device is constituted to receive said prescription data set and store the programming information contained in the prescription data set in said storage device. The processor device of the medical pump device is constituted to load the stored programming information associated with the particular medical fluid from the storage device to carry out an administration operation if identification information read by the reader device relates to the particular medical fluid.

The instant invention implies a change in workflow which may help to improve the ability of a user, in particular a patient, to easily and efficiently program a medical pump device for carrying out an administration operation. Furthermore, an increase in safety may be obtained, reducing the risk for errors in the programming of a medical pump device.

Up to now it has been a potential practice to carry out an administration operation by in a first step reading identification information of an identification mark of a fluid container containing medical fluid using a reader device attached to a medical pump device. According to the identification information, the medical fluid is identified to the medical pump device. The identification information then conventionally is communicated for example to a patient data management system (PDMS) or a prescription software, the patient data management system or prescription software then programming the medical pump device remotely. Upon a check by the user and upon confirming the programming, the administration operation then is started.

In contrast to this conventional concept, by means of the proposed system programming information is transmitted to the medical pump device in a prescription data set that is independent of an actual administration operation to be carried out. The programming information contained in the prescription data set is associated with one or multiple medical fluids and, upon reception at the medical pump device, is stored in the storage device of the medical pump device. Herein, one or multiple prescription data sets relating to one or multiple medical fluids to be administered over an extended course of therapy (for example multiple days) following one or multiple medication protocols relating to one or multiple patients may be transmitted to the medical pump device, the prescription data set(s) and the programming information contained therein being stored in the storage device of the medical pump device in association with the one or multiple medical fluids.

A prescription data set may for example include a full medication protocol for treatment of a specific patient, the medication protocol defining the administration of one or multiple medical fluids for treatment of the patient over an extended duration of therapy, for example over multiple days.

Hence, by transmitting one or multiple prescription data sets to the medical pump device, prescriptions are stored in the medical pump device, the different prescriptions relating to one or multiple different medical fluids, such as medications or enteral feeding solutions.

For initiating an actual administration operation, a user is required to read in, using the reader device of the medical pump device, identification information of an identification mark on a fluid container containing a medical fluid to be administered. By means of the identification information the medical fluid of the fluid container is identified to the medical pump device, upon which the processor device of the medical pump device loads stored programming information associated with the particular medical fluid identified by the identification information and stored in the storage device of the medical pump device. By loading the programming information from the storage device, the medical pump device is programmed, and upon confirmation of the programming settings by a user, for example a patient, and upon connecting the fluid container to the medical pump device, the administration operation is started.

According to the proposed system, hence, a prescription data set comprising programming information relating to one or multiple medical fluids is transmitted to the medical pump device beforehand, the prescription data set for example relating to a medication protocol for an extended therapy for a patient. If identification information relating to a particular medical fluid to which the prescription data set relates is read in by the reader device of the medical pump device, such that the medical pump device recognizes and associates the medical fluid contained in the fluid container with the prescription data set stored in the medical pump device and comprising programming information associated with the medical fluid, the processor device loads programming information relating to the medical fluid and in this way programs the medical pump device. The programming hence takes place automatically according to the stored programming information (which is received at the medical pump device beforehand) upon identifying and recognizing the association of the medical fluid contained in a fluid container with a pre-stored prescription data set.

Because the programming hence takes place automatically, safety is improved, because the risk for errors is decreased.

Furthermore, the programming becomes easy and efficient for the user, because all the user, for example a patient, has to do is to read in identification information on a fluid container using the reader device of the medical pump device. A need for user interactions hence is reduced to a minimum, alleviating in particular the need for a manual entering of programming information.

As a further advantage, with the proposed system the requirements for a connectivity of the medical pump device is reduced. In particular, a connectivity of the medical pump device is required only during transmission and reception of the prescription data set, which may be independent of an actual administration operation. During an actual administration operation, the medical pump device does not have to be connected to a communication network, but can be operated offline.

In one embodiment, the communication device, for example constituted by a server or the like, is constituted to transmit the prescription data set to the medical pump device in a push message. The prescription data set hence is transmitted to the medical pump device by pushing the prescription data set to the medical pump device. The prescription data set hence is transmitted uni-directionally to the medical pump device, without the medical pump device initiating the transmission by a prior request message.

The programming information in principle may include all information relevant for programming a medical pump device for carrying out an administration operation for one or multiple medical fluids within the context of a medication protocol of a patient. The programming information in particular may include a delivery mode (for example continuous infusion or ramp-up infusion), a dose rate, an administration volume and a maximum dose, wherein also other parameters relating to an administration operation may be contained in the programming information. The programming information, in the prescription data set, is related to one or multiple medical fluids and is used, upon identifying one of the medical fluids in a fluid container by means of the reader device, in connection with the identified medical fluid.

The identification mark on the fluid container may for example be a barcode or an RFID tag. Accordingly, the reader device is constituted as a barcode reader or an RFID reader device. In the identification mark identification information relating to the medical fluid contained in the fluid container is encoded and can be read out by means of the reader device upon scanning the identification mark.

In addition, it is conceivable that the identification mark also encodes information relating to the programming of the medical pump device, for example programming information such as the dose rate, an administration volume or a maximum dose.

In addition or alternatively, the identification mark may also encode information relating to the patient, for example information identifying the patient and/or demographic information such as the patient's age, weight, gender and the like.

Within the proposed solution, the communication device communicates a prescription data set (relating to for example a medication protocol defining a therapy for a patient in the course of which one or multiple medical fluids shall be administered to the patient) to the pump device, preferably using a push message. If then, by means of the reader device of the pump device, a medical fluid according to an identification mark is identified and for example additionally patient information included in the identification mark is read in, programming information included in the prescription data set and relating to the identified medical fluid and the patient is loaded into the pump device, such that the actual infusion operation can be started.

Alternatively, instead of beforehand communicating a prescription data set from a communication device such as a server to the pump device, information relating to a medication delivery protocol may also be encoded in the identification mark of a medication container, such that by scanning the identification mark such information may be read into the pump device and may be used for programming the pump. This alternative solution implies that the identification mark is customized for a specific patient to include specific programming information for the administration of the medication contained in the medication container to the patient.

By means of the proposed system the programming of the infusion device may take place automatically without the need for a user interaction for entering programming information. Upon identifying a medical fluid by reading identification information of an identification mark using the reader device, the programming information relating to the medical fluid identified by the identification information is loaded automatically, and in this way the medical pump device is programmed. However, a confirmation of the programming settings by a user, for example a patient, may be required, upon which an administration operation may be started (provided that the fluid container is connected to the medical pump device).

In one embodiment, the reader device may be integral to the medical pump device. In particular, the reader device may be integrated in a housing of the medical pump device and hence does not represent an external device to the medical pump device. The reader device may for example be constituted by a camera suitable to scan a barcode. Or the reader device may include an antenna for coupling to an RFID tag.

The medical pump device may be a portable pump suitable for a homecare use.

The medical pump device may in particular be located at a remote location from the communication device. In particular, a medical pump device may be located at a patient's home, hence remote from a healthcare professional, such as a physician or a nurse in a hospital or in private practice.

One way of providing connection to the medical pump device is via a low power wide area network connection. Low power wide area network (in short LPWAN or LPN) stands for a class of network protocols for connecting low energy devices (for example driven by a battery) to a network server. The protocol is constituted such that a long-range communication at a low energy consumption of, in the instant case, the medical pump devices can be achieved, the communication being secure according to medical device regulations.

A suitable network architecture of a low power wide area network is for example the LoRaWAN (short for Long-Range Wide Area Network), which is an industry standard defined by the LoRa Alliance and making use of LoRa modulation. LoRaWAN allows for a bidirectional communication and has been developed for the Internet of Things (IoT). LoRaWAN uses frequencies around 868 MHz in Europe and around 915 MHz in the US and allows for a communication in a long range, for example up to 40 km.

In principle, also other communication protocols may be used, such as Symphony Link, LTE-M, NarrowBand-loT (NB-loT), Weightless-N, Weightless-P, Weightless-W, or Wi-Fi HaLow, which also represent low power wide are networks.

It is a desire that medical pump devices, such as pumps for the enteral feeding or infusion pumps to provide intravenous infusions, may run on battery for an extended period of time, for example for (at least) 24 hours. A wireless communication with a server hence should take place at low energy without a large impact on the device's battery. For this reason, the connection between the medical pump device and the server is established via a gateway, also denoted as base station, which communicates with the medical pump device wirelessly making use of a low power wide area network connection. Using LoRaWAN, the energy consumption during an active communication may for example be around 10 mA and in an idle mode about 100 nA.

A low power wide area network connection typically allows for a data communication at a rather low data rate. For example, for a LoRaWAN connection the data rate may lie between 0.3 kbps and 50 kbps. This generally may suffice to communicate information between a medical pump device and the gateway to transmit prescription data sets containing programming information.

Although the use of a low power wide area network connection may offer advantages, other network connections for the medical pump device, for example via a public communication network such as the Internet, are applicable and may provide for a connectivity of the medical pump device.

In addition or alternatively, the medical pump device may comprise communication facilities to communicate using the GSM technology, 3G/4G/5G communication technology or the like to communicate with the communication device in particular for receiving one or multiple prescription data sets relating to one or multiple medical fluids in the context of a medication protocol as a patient.

According to an alternative solution, a system for providing homecare to a patient comprises a medical pump device programmable to carry out an administration operation for administering a medical fluid to a patient, the medical pump device comprising a pumping mechanism for administering the medical fluid, a processor device for controlling operation of the pumping mechanism according to the programming information, and a reader device for reading identification information of an identification mark of a fluid container containing medical fluid. Herein, the reader device of the medical pump device is constituted to read, from the identification mark of the fluid container, information relating to a prescription data set comprising programming information associated with the medical fluid contained in the fluid container, wherein the processor device of the medical pump device is constituted to load the programming information associated with the medical fluid to carry out an administration operation.

According to this alternative solution, programming information relating to a medication delivery protocol of a patient is encoded in an identification mark of a medication container and is read into the pump device by means of the reader device upon scanning the identification mark. According to the programming information read from the identification mark, the pump device is programmed for carrying out an administration operation for the medication contained in the medication container.

This implies that the identification mark of the medication container is customized for the patient and the medication to be administered such that the identification mark encodes programming information for the administration of the medication.

The idea underlying the invention shall subsequently be described in more detail with regard to the embodiments shown in the figures. Herein:
- Fig. 1: shows a schematic drawing of a system comprising a medical pump device which is automatically programmable by means of a prescription data set transmitted from a communication device; and
- Fig. 2: shows a flow chart of a workflow for automatically programming a medical pump device.

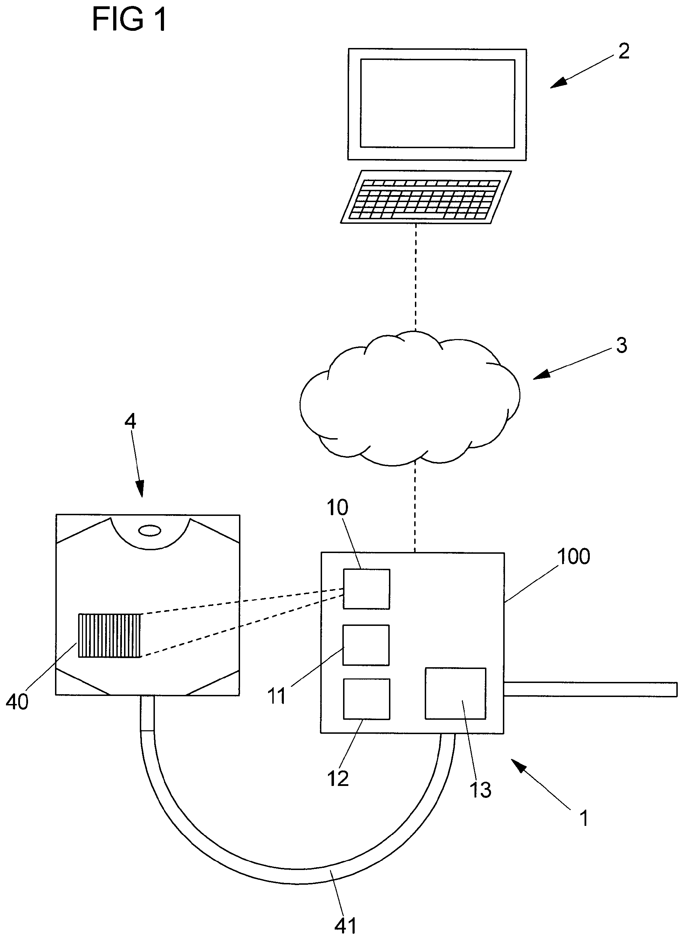

Fig. 1 shows a schematic view of a system comprising a medical pump device 1 to be used in a homecare environment for providing homecare to a patient. The medical pump device 1 may for example be a portable pump comprising a pump mechanism 13 (which only schematically is illustrated in Fig. 1) to which a fluid container 4 may be connected to administer a medical fluid from the fluid container 4 towards a patient.

By means of the medical pump device 1 for example a medication or an enteral feeding solution, such as a nutritional solution, may be administered to a patient. Such medication may for example be an analgesic medication to provide analgesic therapy to a patient in a homecare environment.

The medical pump device 1 furthermore comprises a reader device 10, such as a barcode reader or an RFID reader device, for reading an identification mark 40, such as a barcode or an RFID tag, of a medical fluid container 4. By means of the reader device 10, identification information identifying a medical fluid contained in the medical fluid container 4 and other information contained in the identification mark 40 can be read in such that the medical fluid is identified to the medical pump device 1.

In addition, the medical pump device 1 comprises a processor device 11 and a storage device 12.

For carrying out an administration operation, the medical pump device 1 is to be programmed to define administration parameters such as a dose rate, a dose volume and a mode of administration for a particular medical fluid to be administered. Upon programming the medical pump device 1 and upon connecting the medical fluid container 4 to the medical pump device 1 by means of a suitable infusion line 41, the administration operation can be established, for example by a user confirmation (for example by pressing a dedicated button on the medical pump device 1).

It in the instant context is proposed to automatically program the medical pump device 1 remotely by transmitting a prescription data set comprising programming information from a communication device 2, for example a server remote from the medical pump device 1, to the medical pump device 1 via a suitable network connection established via a communication network 3, such as a public communication network, for example the Internet, or the a low power wide area network.

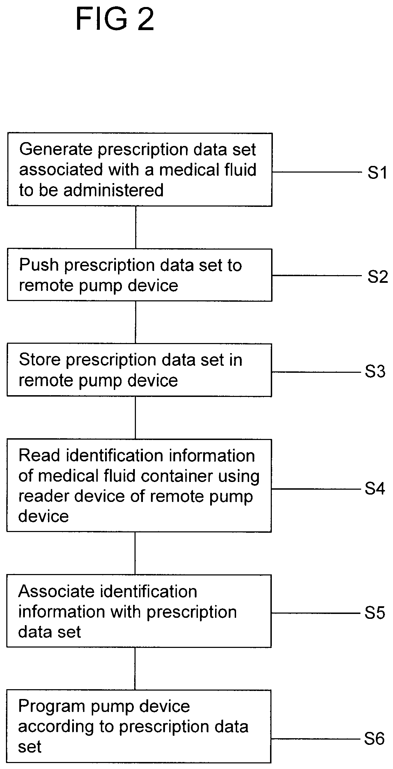

In particular, a workflow as illustrated in the flow diagram of Fig. 2 is proposed.

Within the workflow, at first a prescription data set is generated at the communication device 2, for example by a medical caregiver entering prescription information into the communication device 2, for example a server connected to the communication network 3 (step S1 in Fig. 2). The prescription data set may relate to a medication protocol for the administration of one or multiple medical fluids in the cause of a potentially extended period of therapy to a patient and hence may define a full protocol for therapy of the patient.

Upon generating the prescription data set associated with one or multiple medical fluids to be administered and containing programming information relating to the one or multiple medical fluids, the prescription data set is transmitted to the medical pump device 1 via a communication connection established via the communication connection 3, in particular in a push message in a unidirectional transmission towards the medical pump device 1 (step S2). Upon reception of the push message containing the prescription data set, the medical pump device 1 stores the programming information contained in the prescription data set in the storage device 12 of the medical pump device 1 (step S3).

One or multiple prescription data sets of this kind may be created and may be pushed to the medical pump device 1, such that the medical pump device 1 may store multiple prescription data sets defining prescriptions for administering multiple different medical fluids to a patient, for example in the context of different medication protocols. In the prescription data sets, programming information relating to particular medical fluids is defined, in particular in each case a dose volume, a dose rate and a mode of administration for a particular medical fluid.

The generation and transmission of the prescription data sets may take place independent of an actual administration operation. Push messages containing the prescription data set may be transmitted to the medical pump device 1 for example in an idle mode of the medical pump device 1, i.e., at a time when no administration operations are carried out.

If an actual administration operation shall be carried out, a user, for example a patient, may read in identification information from an identification mark 40 of a fluid container 4 to be used for an administration operation using the reader device 10 (step S4). In this way the medical fluid contained in the fluid container 4 is identified and recognized by the medical pump device 1 (step S5), and if a prescription data set relating to the specific, recognized medical fluid is stored in the storage device, the programming information contained in that prescription data set and relating to the identified medical fluid is loaded by the processor device 11 in order to program the medical pump device 1 (step S6). Hence, by reading the identification information from the identification mark 14 and by in this way identifying the medical fluid to the medical pump device 1, an automatic programming of the medical pump device 1 is initiated, the automatic programming requiring no further user input and in particular no entering of additional data into the medical pump device 1.

Upon connecting the medical fluid container 4 to the medical pump device 1 and upon confirming the automatically loaded programming settings, the administration operation then is started.

A network connection between the medical pump device 1 and the communication device 2 needs to be present only during transmission of the one or the multiple prescription data sets. During an actual administration operation the medical pump device 1 does not have to be connected to the communication device 2 and can be operated offline. The requirements for a connectivity of the medical pump device 1 hence are substantially eased, in particular no connectivity being required for carrying out an actual administration operation.

The medical pump device 1 may for example be a pump for the enteral feeding of a patient or an infusion pump for the intravenous infusion of medical fluids such as medication or the like. A medical pump device 1 may for example be located in a patient's home and may serve to provide homecare to a patient. By means of a medical pump device 1 a patient may autonomously set up and carry out delivery operations, hence allowing the patient to treat himself/herself at home without the presence of a healthcare professional such as a physician or a nurse.

In one embodiment, the medical pump device 1 may communicate with a gateway of the network 3 via a LoRaWAN connection according to the standardized LoRa protocol. LoRaWAN is a low power wide area network (in short LPWAN or LPN) intended for a wireless communication and in particular intended for devices which run of battery and hence should have a low energy consumption. LoRaWAN allows for a bidirectional communication and also offers localization services.

LoRaWAN typically has a star network topology in which gateways serve as a (transparent) bridge relaying data communication between the medical pump device 1 and a central server. The gateways are connected to the server via standard network connections, in particular via a public communication network such as the Internet providing for IP connections between the gateways and the server. LoRaWAN uses data rates in the range from 0.3 kbps to 50 kbps, wherein the data rate may be managed by means of an adaptive data rate (ADR) scheme to maximize battery life of the medical pump device 1 and the overall network capacity.

Instead of a low power wide area network connection, however also a connectivity according to another network technology, such as a GSM or 3G/4G/5G mobile communication technology or via a public communication network such as the Internet, may be provided.

The reader device 10 of the medical pump device 1, as said, may for example be a barcode reader or an RFID reader device. A barcode reader may for example use a camera integrated into a housing 100 of the medical pump device 1, such that the reader device 10 is fully integrated into the medical pump device 1. An RFID reader may for example use an antenna such as a magnetic loop antenna for coupling to an RFID tag, the antenna being integrated into the housing 100.

In an alternative solution, programming information relating to a medication protocol for administering a medication to a patient may also directly be read from the identification mark 40 of the container 4, such that the programming of the medical pump device 1 may take place based on the programming information read in from the identification mark 40 of the container 4. In this case no prescription data set may have to be transmitted beforehand from the communication device 2.

The two approaches are also combinable, such that the programming of the medical pump device 1 may take place based on programming information contained in a prescription data set received from the communication device 2 and in addition according to programming information encoded in the identification mark 40 of the container 4 and read in from the identification mark 40 upon scanning by the reader device 10.

The idea underlying the invention is not limited to the embodiments described above.

The medical pump device may be constituted in different fashions, for example by a volumetric pump device or a syringe pump device. By using a volumetric pump a medical fluid is delivered from a fluid container such as a flexible bag. By means of a syringe pump a medical fluid is delivered from a fluid container in the shape of a barrel of a syringe.

By transmitting prescription data sets including programming information relating to particular medical fluids beforehand to a medical pump device, an easy, automatic programming of the infusion pump with minimum user interaction becomes possible. This helps to reduce errors in the programming and hence to improve the safety for carrying out administration operations, in particular in a homecare environment.

### List of Reference Numerals

- 1: Medical pump device
- 10: Reader device
- 100: Housing
- 11: Processor device
- 12: Storage device
- 13: Pump mechanism
- 2: Communication network
- 3: Communication device
- 4: Fluid container
- 40: Identification mark (bar code, RFIF tag)
- 41: Administration line
- S1-S6: Steps

## Claims

1. System for providing homecare to a patient, remote from a healthcare professional, allowing the patient to treat himself/herself at home without the presence of a healthcare professional such as a physician or a nurse, the system comprising:
- a medical pump device (1) programmable to carry out an administration operation for administering a medical fluid to a patient, the medical pump device (1) comprising a pumping mechanism (13) for administering the medical fluid, a storage device (12) for storing programming information, a processor device (11) for controlling operation of the pumping mechanism (13) according to the programming information, and a reader device (10) for reading identification information of an identification mark (40) of a fluid container (4) containing medical fluid, and
- a communication device (2) being in communication connection with the medical pump device (1) via a communication network (3),
wherein the system is configured so that the communication device (2) is constituted to transmit a prescription data set comprising programming information associated with a particular medical fluid to the medical pump device (1), in a push message, without the medical pump device initiating the transmission by a prior request message, and the medical pump device (1) is constituted to receive said prescription data set and store the programming information contained in the prescription data set in said storage device (12), wherein the processor device (11) of the medical pump device (1) is constituted to load the stored programming information associated with the particular medical fluid from the storage device (12) to carry out an administration operation if identification information read by the reader device (10) relates to the particular medical fluid,
and in that the medical pump device (1) communicates with a gateway of the communication network (3) via a LoRaWAN connection according to the standardized LoRa protocol.

2. System for providing homecare to a patient, remote from a healthcare professional, allowing the patient to treat himself/herself at home without the presence of a healthcare professional such as a physician or a nurse, the system comprising:
- a medical pump device (1) programmable to carry out an administration operation for administering a medical fluid to a patient, the medical pump device (1) comprising a pumping mechanism (13) for administering the medical fluid, a storage device (12) for storing programming information, a processor device (11) for controlling operation of the pumping mechanism (13) according to the programming information, and a reader device (10) for reading identification information of an identification mark (40) of a fluid container (4) containing medical fluid, and
- a communication device (2) being in communication connection with the medical pump device (1) via a communication network (3),
wherein the system is configured so that the communication device (2) is constituted to transmit a prescription data set comprising programming information associated with a particular medical fluid to the medical pump device (1), in a push message, without the medical pump device initiating the transmission by a prior request message, and the medical pump device (1) is constituted to receive said prescription data set and store the programming information contained in the prescription data set in said storage device (12), wherein the processor device (11) of the medical pump device (1) is constituted to load the stored programming information associated with the particular medical fluid from the storage device (12) to carry out an administration operation if identification information read by the reader device (10) relates to the particular medical fluid,
and in that the medical pump device (1) communicates with a gateway of the communication network (3) via GSM or 3G/4G/5G mobile technology or via a public communication network such as the internet.

3. System according to one of the preceding claims, **characterized in that** the programming information comprises at least one of information relating to a delivery mode, a dose rate, an administration volume, and a maximum dose.

4. System according to one of the preceding claims, **characterized in that** the identification mark (40) is a barcode or an RFID tag.

5. System according to one of the preceding claims, **characterized in that** the processor device (11) is constituted to start an administration operation according to the loaded programming information upon confirmation by a user.

6. System according to one of the preceding claims, **characterized in that** the reader device (10) is embedded in a housing (100) of the medical pump device (1).

7. System according to one of the preceding claims, **characterized in that** the medical pump device (1) is located at a remote location from the communication device (2).

8. System for providing homecare to a patient, remote from a healthcare professional, allowing the patient to treat himself/herself at home without the presence of a healthcare professional such as a physician or a nurse, the system comprising:
- a medical pump device (1) programmable to carry out an administration operation for administering a medical fluid to a patient, the medical pump device (1) comprising a pumping mechanism (13) for administering the medical fluid, a storage device (12) for storing programming information, a processor device (11) for controlling operation of the pumping mechanism (13) according to the programming information, and a reader device (10) for reading identification information of an identification mark (40) of a fluid container (4) containing medical fluid, and a communication device (2) being in communication connection with the medical pump device (1) via a communication network (3),
wherein the system is configured so that the reader device (10) of the medical pump device (1) is constituted to read, from the identification mark (40) of the fluid container (4), information relating to a prescription data set comprising programming information associated with the medical fluid contained in the fluid container (4), wherein the processor device (11) of the medical pump device (1) is constituted to load the programming information associated with the medical fluid to carry out an administration operation,
and in that the communication device (2) is constituted to transmit a prescription data set comprising programming information associated with a particular medical fluid to the medical pump device (1), in a push message, without the medical pump device initiating the transmission by a prior request message, and the medical pump device (1) is constituted to receive said prescription data set and store the programming information contained in the prescription data set in said storage device (12), wherein the processor device (11) of the medical pump device (1) is constituted to load the stored programming information associated with the particular medical fluid from the storage device (12) to carry out an administration operation if identification information read by the reader device (10) relates to the particular medical fluid,
and in that the medical pump device (1) communicates with a gateway of the communication network (3) via a LoRaWAN connection according to the standardized LoRa protocol.

9. System for providing homecare to a patient, remote from a healthcare professional, allowing the patient to treat himself/herself at home without the presence of a healthcare professional such as a physician or a nurse, the system comprising:
- a medical pump device (1) programmable to carry out an administration operation for administering a medical fluid to a patient, the medical pump device (1) comprising a pumping mechanism (13) for administering the medical fluid, a storage device (12) for storing programming information, a processor device (11) for controlling operation of the pumping mechanism (13) according to the programming information, and a reader device (10) for reading identification information of an identification mark (40) of a fluid container (4) containing medical fluid, and a communication device (2) being in communication connection with the medical pump device (1) via a communication network (3),
wherein the system is configured so that the reader device (10) of the medical pump device (1) is constituted to read, from the identification mark (40) of the fluid container (4), information relating to a prescription data set comprising programming information associated with the medical fluid contained in the fluid container (4), wherein the processor device (11) of the medical pump device (1) is constituted to load the programming information associated with the medical fluid to carry out an administration operation,
and in that the communication device (2) is constituted to transmit a prescription data set comprising programming information associated with a particular medical fluid to the medical pump device (1), in a push message, without the medical pump device initiating the transmission by a prior request message, and the medical pump device (1) is constituted to receive said prescription data set and store the programming information contained in the prescription data set in said storage device (12), wherein the processor device (11) of the medical pump device (1) is constituted to load the stored programming information associated with the particular medical fluid from the storage device (12) to carry out an administration operation if identification information read by the reader device (10) relates to the particular medical fluid,
and in that the medical pump device (1) communicates with a gateway of the communication network (3) via GSM or 3G/4G/5G mobile technology or via a public communication network such as the internet.

## Patentansprüche

1. System zum Bereitstellen von Heimversorgung für einen Patienten/eine Patientin, der/die sich entfernt von einer Gesundheitsfachkraft befindet, wobei der Patient/die Patientin sich zu Hause ohne die Anwesenheit einer Gesundheitsfachkraft wie einer ärztlichen Fachperson oder einer Krankenpflegeperson selbst behandeln kann, wobei das System Folgendes umfasst:
- eine medizinische Pumpvorrichtung (1), die dazu programmierbar ist, eine Verabreichungsoperation zum Verabreichen eines medizinischen Fluids an einen Patienten/eine Patientin durchzuführen, wobei die medizinische Pumpvorrichtung (1) einen Pumpmechanismus (13) zum Verabreichen des medizinischen Fluids, eine Speichervorrichtung (12) zum Speichern von Programmierinformationen, eine Prozessorvorrichtung (11) zum Steuern der Operation des Pumpmechanismus (13) gemäß den Programmierinformationen und eine Lesevorrichtung (10) zum Lesen von Identifikationsinformationen einer Identifikationsmarkierung (40) eines Fluidbehälters (4), der medizinisches Fluid enthält, umfasst, und
- eine Kommunikationsvorrichtung (2), die über ein Kommunikationsnetzwerk (3) mit der medizinischen Pumpvorrichtung (1) in Kommunikationsverbindung steht,
wobei das System derart konfiguriert ist, dass die Kommunikationsvorrichtung (2) dazu ausgelegt ist, einen Verschreibungsdatensatz, der Programmierinformationen umfasst, die einem bestimmten medizinischen Fluid zugeordnet sind, in einer Push-Nachricht an die medizinische Pumpvorrichtung (1) zu übertragen, ohne dass die medizinische Pumpvorrichtung die Übertragung durch eine vorherige Anforderungsnachricht initiiert, und die medizinische Pumpvorrichtung (1) dazu ausgelegt ist, den Verschreibungsdatensatz zu empfangen und die in dem Verschreibungsdatensatz enthaltenen Programmierinformationen in der Speichervorrichtung (12) zu speichern, wobei die Prozessorvorrichtung (11) der medizinischen Pumpvorrichtung (1) dazu ausgelegt ist, die gespeicherten Programmierinformationen, die dem bestimmten medizinischen Fluid zugeordnet sind, aus der Speichervorrichtung (12) zu laden, um eine Verabreichungsoperation durchzuführen, falls sich die von der Lesevorrichtung (10) gelesenen Identifikationsinformationen auf das bestimmte medizinische Fluid beziehen,
und dass die medizinische Pumpvorrichtung (1) über eine LoRaWAN-Verbindung gemäß dem standardisierten LoRa-Protokoll mit einem Gateway des Kommunikationsnetzwerks (3) kommuniziert.

2. System zum Bereitstellen von Heimversorgung für einen Patienten/eine Patientin, der/die sich entfernt von einer Gesundheitsfachkraft befindet, wobei der Patient/die Patientin sich zu Hause ohne die Anwesenheit einer Gesundheitsfachkraft wie einer ärztlichen Fachperson oder einer Krankenpflegeperson selbst behandeln kann, wobei das System Folgendes umfasst:
- eine medizinische Pumpvorrichtung (1), die dazu programmierbar ist, eine Verabreichungsoperation zum Verabreichen eines medizinischen Fluids an einen Patienten/eine Patientin durchzuführen, wobei die medizinische Pumpvorrichtung (1) einen Pumpmechanismus (13) zum Verabreichen des medizinischen Fluids, eine Speichervorrichtung (12) zum Speichern von Programmierinformationen, eine Prozessorvorrichtung (11) zum Steuern der Operation des Pumpmechanismus (13) gemäß den Programmierinformationen und eine Lesevorrichtung (10) zum Lesen von Identifikationsinformationen einer Identifikationsmarkierung (40) eines Fluidbehälters (4), der medizinisches Fluid enthält, umfasst, und
- eine Kommunikationsvorrichtung (2), die über ein Kommunikationsnetzwerk (3) mit der medizinischen Pumpvorrichtung (1) in Kommunikationsverbindung steht,
wobei das System derart konfiguriert ist, dass die Kommunikationsvorrichtung (2) dazu ausgelegt ist, einen Verschreibungsdatensatz, der Programmierinformationen umfasst, die einem bestimmten medizinischen Fluid zugeordnet sind, in einer Push-Nachricht an die medizinische Pumpvorrichtung (1) zu übertragen, ohne dass die medizinische Pumpvorrichtung die Übertragung durch eine vorherige Anforderungsnachricht initiiert, und die medizinische Pumpvorrichtung (1) dazu ausgelegt ist, den Verschreibungsdatensatz zu empfangen und die in dem Verschreibungsdatensatz enthaltenen Programmierinformationen in der Speichervorrichtung (12) zu speichern, wobei die Prozessorvorrichtung (11) der medizinischen Pumpvorrichtung (1) dazu ausgelegt ist, die gespeicherten Programmierinformationen, die dem bestimmten medizinischen Fluid zugeordnet sind, aus der Speichervorrichtung (12) zu laden, um eine Verabreichungsoperation durchzuführen, falls sich die von der Lesevorrichtung (10) gelesenen Identifikationsinformationen auf das bestimmte medizinische Fluid beziehen,
und dass die medizinische Pumpvorrichtung (1) über GSM- oder 3G/4G/5G-Mobiltechnologie oder über ein öffentliches Kommunikationsnetzwerk wie das Internet mit einem Gateway des Kommunikationsnetzwerks (3) kommuniziert.

3. System nach einem der vorhergehenden Ansprüche, **dadurch gekennzeichnet, dass** die Programmierinformationen mindestens eine von Informationen bezüglich eines Verabreichungsmodus, einer Dosisrate, eines Verabreichungsvolumens und einer Maximaldosis umfassen.

4. System nach einem der vorhergehenden Ansprüche, **dadurch gekennzeichnet, dass** die Identifikationsmarkierung (40) ein Strichcode oder ein RFID-Tag ist.

5. System nach einem der vorhergehenden Ansprüche, **dadurch gekennzeichnet, dass** die Prozessorvorrichtung (11) dazu ausgelegt ist, nach Bestätigung durch einen Benutzer eine Verabreichungsoperation gemäß den geladenen Programmierinformationen zu starten.

6. System nach einem der vorhergehenden Ansprüche, **dadurch gekennzeichnet, dass** die Lesevorrichtung (10) in ein Gehäuse (100) der medizinischen Pumpvorrichtung (1) eingebettet ist.

7. System nach einem der vorhergehenden Ansprüche, **dadurch gekennzeichnet, dass** sich die medizinische Pumpvorrichtung (1) an einem von der Kommunikationsvorrichtung (2) entfernten Standort befindet.

8. System zum Bereitstellen von Heimversorgung für einen Patienten/eine Patientin, der/die sich entfernt von einer Gesundheitsfachkraft befindet, wobei der Patient/die Patientin sich zu Hause ohne die Anwesenheit einer Gesundheitsfachkraft wie einer ärztlichen Fachperson oder einer Krankenpflegeperson selbst behandeln kann, wobei das System Folgendes umfasst:
- eine medizinische Pumpvorrichtung (1), die dazu programmierbar ist, eine Verabreichungsoperation zum Verabreichen eines medizinischen Fluids an einen Patienten/eine Patientin durchzuführen, wobei die medizinische Pumpvorrichtung (1) einen Pumpmechanismus (13) zum Verabreichen des medizinischen Fluids, eine Speichervorrichtung (12) zum Speichern von Programmierinformationen, eine Prozessorvorrichtung (11) zum Steuern der Operation des Pumpmechanismus (13) gemäß den Programmierinformationen und eine Lesevorrichtung (10) zum Lesen von Identifikationsinformationen einer Identifikationsmarkierung (40) eines Fluidbehälters (4), der medizinisches Fluid enthält, umfasst, und eine Kommunikationsvorrichtung (2), die über ein Kommunikationsnetzwerk (3) mit der medizinischen Pumpvorrichtung (1) in Kommunikationsverbindung steht,
wobei das System derart konfiguriert ist, dass die Lesevorrichtung (10) der medizinischen Pumpvorrichtung (1) dazu ausgelegt ist, aus der Identifikationsmarkierung (40) des Fluidbehälters (4) Informationen bezüglich eines Verschreibungsdatensatzes zu lesen, der Programmierinformationen umfasst, die dem in dem Fluidbehälter (4) enthaltenen medizinischen Fluid zugeordnet sind, wobei die Prozessorvorrichtung (11) der medizinischen Pumpvorrichtung (1) dazu ausgelegt ist, die dem medizinischen Fluid zugeordneten Programmierinformationen zu laden, um eine Verabreichungsoperation durchzuführen,
und dass die Kommunikationsvorrichtung (2) dazu ausgelegt ist, einen Verschreibungsdatensatz, der Programmierinformationen umfasst, die einem bestimmten medizinischen Fluid zugeordnet sind, in einer Push-Nachricht an die medizinische Pumpvorrichtung (1) zu übertragen, ohne dass die medizinische Pumpvorrichtung die Übertragung durch eine vorherige Anforderungsnachricht initiiert, und die medizinische Pumpvorrichtung (1) dazu ausgelegt ist, den Verschreibungsdatensatz zu empfangen und die in dem Verschreibungsdatensatz enthaltenen Programmierinformationen in der Speichervorrichtung (12) zu speichern, wobei die Prozessorvorrichtung (11) der medizinischen Pumpvorrichtung (1) dazu ausgelegt ist, die gespeicherten Programmierinformationen, die dem bestimmten medizinischen Fluid zugeordnet sind, aus der Speichervorrichtung (12) zu laden, um eine Verabreichungsoperation durchzuführen, falls sich die von der Lesevorrichtung (10) gelesenen Identifikationsinformationen auf das bestimmte medizinische Fluid beziehen,
und dass die medizinische Pumpvorrichtung (1) über eine LoRaWAN-Verbindung gemäß dem standardisierten LoRa-Protokoll mit einem Gateway des Kommunikationsnetzwerks (3) kommuniziert.

9. System zum Bereitstellen von Heimversorgung für einen Patienten/eine Patientin, der/die sich entfernt von einer Gesundheitsfachkraft befindet, wobei der Patient/die Patientin sich zu Hause ohne die Anwesenheit einer Gesundheitsfachkraft wie einer ärztlichen Fachperson oder einer Krankenpflegeperson selbst behandeln kann, wobei das System Folgendes umfasst:
- eine medizinische Pumpvorrichtung (1), die dazu programmierbar ist, eine Verabreichungsoperation zum Verabreichen eines medizinischen Fluids an einen Patienten/eine Patientin durchzuführen, wobei die medizinische Pumpvorrichtung (1) einen Pumpmechanismus (13) zum Verabreichen des medizinischen Fluids, eine Speichervorrichtung (12) zum Speichern von Programmierinformationen, eine Prozessorvorrichtung (11) zum Steuern der Operation des Pumpmechanismus (13) gemäß den Programmierinformationen und eine Lesevorrichtung (10) zum Lesen von Identifikationsinformationen einer Identifikationsmarkierung (40) eines Fluidbehälters (4), der medizinisches Fluid enthält, umfasst, und eine Kommunikationsvorrichtung (2), die über ein Kommunikationsnetzwerk (3) mit der medizinischen Pumpvorrichtung (1) in Kommunikationsverbindung steht,
wobei das System derart konfiguriert ist, dass die Lesevorrichtung (10) der medizinischen Pumpvorrichtung (1) dazu ausgelegt ist, aus der Identifikationsmarkierung (40) des Fluidbehälters (4) Informationen bezüglich eines Verschreibungsdatensatzes zu lesen, der Programmierinformationen umfasst, die dem in dem Fluidbehälter (4) enthaltenen medizinischen Fluid zugeordnet sind, wobei die Prozessorvorrichtung (11) der medizinischen Pumpvorrichtung (1) dazu ausgelegt ist, die dem medizinischen Fluid zugeordneten Programmierinformationen zu laden, um eine Verabreichungsoperation durchzuführen,
und dass die Kommunikationsvorrichtung (2) dazu ausgelegt ist, einen Verschreibungsdatensatz, der Programmierinformationen umfasst, die einem bestimmten medizinischen Fluid zugeordnet sind, in einer Push-Nachricht an die medizinische Pumpvorrichtung (1) zu übertragen, ohne dass die medizinische Pumpvorrichtung die Übertragung durch eine vorherige Anforderungsnachricht initiiert, und die medizinische Pumpvorrichtung (1) dazu ausgelegt ist, den Verschreibungsdatensatz zu empfangen und die in dem Verschreibungsdatensatz enthaltenen Programmierinformationen in der Speichervorrichtung (12) zu speichern, wobei die Prozessorvorrichtung (11) der medizinischen Pumpvorrichtung (1) dazu ausgelegt ist, die gespeicherten Programmierinformationen, die dem bestimmten medizinischen Fluid zugeordnet sind, aus der Speichervorrichtung (12) zu laden, um eine Verabreichungsoperation durchzuführen, falls sich die von der Lesevorrichtung (10) gelesenen Identifikationsinformationen auf das bestimmte medizinische Fluid beziehen,
und dass die medizinische Pumpvorrichtung (1) über GSM- oder 3G/4G/5G-Mobiltechnologie oder über ein öffentliches Kommunikationsnetzwerk wie das Internet mit einem Gateway des Kommunikationsnetzwerks (3) kommuniziert.

## Revendications

1. Système de soins à domicile pour un patient ou une patiente, à distance par un professionnel de la santé, permettant au patient ou à la patiente de se soigner à domicile sans la présence d'un professionnel de santé tel qu'un médecin ou un infirmier, le système comprenant :
- un dispositif de pompe médicale (1) programmable pour effectuer une opération d'administration destinée à administrer un fluide médical à un patient ou à une patiente, le dispositif de pompe médicale (1) comprenant un mécanisme de pompage (13) destiné à administrer le fluide médical, un dispositif de stockage (12) destiné à stocker des informations de programmation, un dispositif de traitement (11) destiné à commander le fonctionnement du mécanisme de pompage (13) en fonction des informations de programmation, et un dispositif de lecture (10) destiné à lire des informations d'identification d'une marque d'identification (40) d'un réservoir de fluide (4) contenant un fluide médical, et
- un dispositif de communication (2) étant en liaison de communication avec le dispositif de pompe médicale (1) par l'intermédiaire d'un réseau de communication (3),
dans lequel le système est configuré de sorte que le dispositif de communication (2) est constitué pour transmettre un ensemble de données de prescription comprenant des informations de programmation associées à un fluide médical particulier au dispositif de pompe médicale (1), dans un message push, sans que le dispositif de pompe médicale ne déclenche la transmission par un message de demande préalable, et le dispositif de pompe médicale (1) est constitué pour recevoir ledit ensemble de données de prescription et stocker les informations de programmation contenues dans l'ensemble de données de prescription dans ledit dispositif de stockage (12), dans lequel le dispositif de traitement (11) du dispositif de pompe médicale (1) est constitué pour charger les informations de programmation stockées associées au fluide médical particulier à partir du dispositif de stockage (12) pour effectuer une opération d'administration si les informations d'identification lues par le dispositif de lecture (10) se rapportent au fluide médical particulier,
et en ce que le dispositif de pompe médicale (1) communique avec une passerelle du réseau de communication (3) par l'intermédiaire d'une connexion LoRaWAN conformément au protocole LoRa normalisé.

2. Système de soins à domicile pour un patient ou une patiente, à distance par un professionnel de la santé, permettant au patient ou à la patiente de se soigner à domicile sans la présence d'un professionnel de santé tel qu'un médecin ou un infirmier, le système comprenant :
- un dispositif de pompe médicale (1) programmable pour effectuer une opération d'administration destinée à administrer un fluide médical à un patient ou à une patiente, le dispositif de pompe médicale (1) comprenant un mécanisme de pompage (13) destiné à administrer le fluide médical, un dispositif de stockage (12) destiné à stocker des informations de programmation, un dispositif de traitement (11) destiné à commander le fonctionnement du mécanisme de pompage (13) en fonction des informations de programmation, et un dispositif de lecture (10) destiné à lire des informations d'identification d'une marque d'identification (40) d'un réservoir de fluide (4) contenant un fluide médical, et
- un dispositif de communication (2) étant en liaison de communication avec le dispositif de pompe médicale (1) par l'intermédiaire d'un réseau de communication (3),
dans lequel le système est configuré de sorte que le dispositif de communication (2) est constitué pour transmettre un ensemble de données de prescription comprenant des informations de programmation associées à un fluide médical particulier au dispositif de pompe médicale (1), dans un message push, sans que le dispositif de pompe médicale ne déclenche la transmission par un message de demande préalable, et le dispositif de pompe médicale (1) est constitué pour recevoir ledit ensemble de données de prescription et stocker les informations de programmation contenues dans l'ensemble de données de prescription dans ledit dispositif de stockage (12), dans lequel le dispositif de traitement (11) du dispositif de pompe médicale (1) est constitué pour charger les informations de programmation stockées associées au fluide médical particulier à partir du dispositif de stockage (12) pour effectuer une opération d'administration si les informations d'identification lues par le dispositif de lecture (10) se rapportent au fluide médical particulier,
et en ce que le dispositif de pompe médicale (1) communique avec une passerelle du réseau de communication (3) par l'intermédiaire de la technologie mobile GSM ou 3G/4G/5G ou par l'intermédiaire d'un réseau de communication public tel qu'internet.

3. Système selon l'une des revendications précédentes, **caractérisé en ce que** les informations de programmation comprennent au moins l'une des informations relatives à un mode de distribution, à un débit de dose, à un volume d'administration, et à une dose maximale.

4. Système selon l'une des revendications précédentes, **caractérisé en ce que** la marque d'identification (40) est un code-barres ou une étiquette RFID.

5. Système selon l'une des revendications précédentes, **caractérisé en ce que** le dispositif de traitement (11) est constitué pour démarrer une opération d'administration conformément aux informations de programmation chargées sur confirmation d'un utilisateur.

6. Système selon l'une des revendications précédentes, **caractérisé en ce que** le dispositif de lecture (10) est intégré dans un boîtier (100) du dispositif de pompe médicale (1).

7. Système selon l'une des revendications précédentes, **caractérisé en ce que** le dispositif de pompe médicale (1) est situé à un emplacement à distance du dispositif de communication (2).

8. Système de soins à domicile pour un patient ou une patiente, à distance par un professionnel de la santé, permettant au patient ou à la patiente de se soigner à domicile sans la présence d'un professionnel de santé tel qu'un médecin ou un infirmier, le système comprenant :
- un dispositif de pompe médicale (1) programmable pour effectuer une opération d'administration destinée à administrer un fluide médical à un patient ou à une patiente, le dispositif de pompe médicale (1) comprenant un mécanisme de pompage (13) destiné à administrer le fluide médical, un dispositif de stockage (12) destiné à stocker des informations de programmation, un dispositif de traitement (11) destiné à commander le fonctionnement du mécanisme de pompage (13) en fonction des informations de programmation, et un dispositif de lecture (10) destiné à lire des informations d'identification d'une marque d'identification (40) d'un réservoir de fluide (4) contenant un fluide médical, et un dispositif de communication (2) étant en liaison de communication avec le dispositif de pompe médicale (1) par l'intermédiaire d'un réseau de communication (3),
dans lequel le système est configuré de sorte que le dispositif de lecture (10) du dispositif de pompe médicale (1) est constitué pour lire, à partir de la marque d'identification (40) du réservoir de fluide (4), des informations relatives à un ensemble de données de prescription comprenant des informations de programmation associées au fluide médical contenu dans le réservoir de fluide (4), dans lequel le dispositif de traitement (11) du dispositif de pompe médicale (1) est constitué pour charger les informations de programmation associées au fluide médical pour effectuer une opération d'administration,
et en ce que le dispositif de communication (2) est constitué pour transmettre un ensemble de données de prescription comprenant des informations de programmation associées à un fluide médical particulier au dispositif de pompe médicale (1), dans un message push, sans que le dispositif de pompe médicale ne déclenche la transmission par un message de demande préalable, et le dispositif de pompe médicale (1) est constitué pour recevoir ledit ensemble de données de prescription et stocker les informations de programmation contenues dans l'ensemble de données de prescription dans ledit dispositif de stockage (12), dans lequel le dispositif de traitement (11) du dispositif de pompe médicale (1) est constitué pour charger les informations de programmation stockées associées au fluide médical particulier à partir du dispositif de stockage (12) pour effectuer une opération d'administration si les informations d'identification lues par le dispositif de lecture (10) se rapportent au fluide médical particulier,
et en ce que le dispositif de pompe médicale (1) communique avec une passerelle du réseau de communication (3) par l'intermédiaire d'une connexion LoRaWAN conformément au protocole LoRa normalisé.

9. Système de soins à domicile pour un patient ou une patiente, à distance par un professionnel de la santé, permettant au patient ou à la patiente de se soigner à domicile sans la présence d'un professionnel de santé tel qu'un médecin ou un infirmier, le système comprenant :
- un dispositif de pompe médicale (1) programmable pour effectuer une opération d'administration destinée à administrer un fluide médical à un patient ou à une patiente, le dispositif de pompe médicale (1) comprenant un mécanisme de pompage (13) destiné à administrer le fluide médical, un dispositif de stockage (12) destiné à stocker des informations de programmation, un dispositif de traitement (11) destiné à commander le fonctionnement du mécanisme de pompage (13) en fonction des informations de programmation, et un dispositif de lecture (10) destiné à lire des informations d'identification d'une marque d'identification (40) d'un réservoir de fluide (4) contenant un fluide médical, et un dispositif de communication (2) étant en liaison de communication avec le dispositif de pompe médicale (1) par l'intermédiaire d'un réseau de communication (3),
dans lequel le système est configuré de sorte que le dispositif de lecture (10) du dispositif de pompe médicale (1) est constitué pour lire, à partir de la marque d'identification (40) du réservoir de fluide (4), des informations relatives à un ensemble de données de prescription comprenant des informations de programmation associées au fluide médical contenu dans le réservoir de fluide (4), dans lequel le dispositif de traitement (11) du dispositif de pompe médicale (1) est constitué pour charger les informations de programmation associées au fluide médical pour effectuer une opération d'administration,
et en ce que le dispositif de communication (2) est constitué pour transmettre un ensemble de données de prescription comprenant des informations de programmation associées à un fluide médical particulier au dispositif de pompe médicale (1), dans un message push, sans que le dispositif de pompe médicale ne déclenche la transmission par un message de demande préalable, et le dispositif de pompe médicale (1) est constitué pour recevoir ledit ensemble de données de prescription et stocker les informations de programmation contenues dans l'ensemble de données de prescription dans ledit dispositif de stockage (12), dans lequel le dispositif de traitement (11) du dispositif de pompe médicale (1) est constitué pour charger les informations de programmation stockées associées au fluide médical particulier à partir du dispositif de stockage (12) pour effectuer une opération d'administration si les informations d'identification lues par le dispositif de lecture (10) se rapportent au fluide médical particulier,
et en ce que le dispositif de pompe médicale (1) communique avec une passerelle du réseau de communication (3) par l'intermédiaire de la technologie mobile GSM ou 3G/4G/5G ou par l'intermédiaire d'un réseau de communication public tel qu'internet.
